# FASCICULE DE BREVET EUROPEEN

(11) **EP 3 505 634 B1**
(45) Date de publication et mention de la délivrance du brevet: **31.03.2021**
(21) Numéro de dépôt: 18185874.7
(22) Date de dépôt: 13.06.2013
(51) Int. Cl.: C12N 15/82, C12N 15/09

(54) **METHODE DE PRODUCTION D'ALLERGENES RECOMBINANTS DE HAUTE QUALITE DANS UNE PLANTE**
METHODE ZUR ERZEUGUNG VON REKOMBINANTEN ALLERGENEN HOHER QUALITÄT IN EINER PFLANZE
METHOD FOR PRODUCING HIGH-QUALITY RECOMBINANT ALLERGENS IN A PLANT

(30) Priorité: 13.06.2012 FR 1255510
(43) Date de publication de la demande: 03.07.2019
(62) Demande divisionnaire de: 13733399.3
(73) Titulaire: Angany Inc., Québec, QC G1R 1R2 (CA)
(72) Inventeur: GOMORD, Véronique, 76000 Rouen (FR); FITCHETTE, Anne Catherine, 76570 Mesnil Panneville (FR); FAYE, Loïc, 76160 ST Jacques Sur Darnetal (FR)
(74) Mandataire: Plasseraud IP

(56) Documents cités:
- WO-A2-2008/056265
- US-A1- 2006 077 634
- VÉZINA LOUIS-P ET AL: "Transient co-expression for fast and high-yield production of antibodies with human-like N-glycans in plants", PLANT BIOTECHNOLOGY JOURNAL, BLACKWELL, OXFORD, GB, vol. 7, no. 5, 1 juin 2009 (2009-06-01), pages 442-455, XP002582248, ISSN: 1467-7644, DOI: 10.1111/J.1467-7652.2009.00414.X [extrait le 2009-04-21]
- OBERMEYER G ET AL: "Over-expression and production of plant allergens by molecular farming strategies", METHODS : A COMPANION TO METHODS IN ENZYMOLOGY, ACADEMIC PRESS INC., NEW YORK, NY, US, vol. 32, no. 3, 1 mars 2004 (2004-03-01), pages 235-240, XP004488973, ISSN: 1046-2023, DOI: 10.1016/J.YMETH.2003.08.012
- LUCA SANTI ET AL: "Protection conferred by recombinant Yersinia pestis antigens produced by a rapid and highly scalable plant expression system", PROCEEDINGS OF THE NATIONAL ACADEMY OF SCIENCES, NATIONAL ACADEMY OF SCIENCES, US, vol. 103, no. 4, 24 janvier 2006 (2006-01-24), pages 861-866, XP008129743, ISSN: 0027-8424, DOI: 10.1073/PNAS.0510014103
- OLAWOLE O OBEMBE ET AL: "Advances in plant molecular farming", BIOTECHNOLOGY ADVANCES, ELSEVIER PUBLISHING, BARKING, GB, vol. 29, no. 2, 12 novembre 2010 (2010-11-12), pages 210-222, XP028136133, ISSN: 0734-9750, DOI: 10.1016/J.BIOTECHADV.2010.11.004 [extrait le 2010-11-27]
- Y GLEBA ET AL: "Magnifection?a new platform for expressing recombinant vaccines in plants", VACCINE, vol. 23, no. 17-18, 18 mars 2005 (2005-03-18), pages 2042-2048, XP055029853, ISSN: 0264-410X, DOI: 10.1016/j.vaccine.2005.01.006
- MUSIYCHUK KONSTANTIN ET AL: "A launch vector for the production of vaccine antigens in plants", INFLUENZA AND OTHER RESPIRATORY VIRUSES, BLACKWELL PUBLISHING LTD, UK, vol. 1, no. 1, 1 janvier 2007 (2007-01-01) , pages 19-25, XP009058889, ISSN: 1750-2640, DOI: 10.1111/J.1750-2659.2006.00005.X [extrait le 2007-01-19]
- BENOIT DE MUYNCK ET AL: "Production of antibodies in plants: status after twenty years", PLANT BIOTECHNOLOGY JOURNAL, vol. 8, no. 5, 3 février 2010 (2010-02-03) , pages 529-563, XP055038516, ISSN: 1467-7644, DOI: 10.1111/j.1467-7652.2009.00494.x

### SEQUENCE LISTING

<110> ANGANY GENETICS
<120> Méthode de production d'allergènes recombinants de haute qualité par expression transitoire chez Nicotiana benthamiana
<130> BCT130167 QT
<160> 21
<170> PatentIn version 3.5
<210> 1
   <211> 13
   <212> PRT
   <213> Artificial Sequence
<220>
   <223> signal peptide
<400> 1
   Met Thr Gly Ala Ser Arg Arg Ser Ala Arg Gly Arg Ile
   1               5                   10
<210> 2
   <211> 10
   <212> PRT
   <213> Artificial Sequence
<220>
   <223> signal peptide
<400> 2
   Met Ala Arg Gly Glu Arg Arg Arg Arg Ala
   1 5 10
<210> 3
   <211> 12
   <212> PRT
   <213> Artificial Sequence
<220>
   <223> signal peptide
<400> 3
   Met Asn Asp Arg Arg Pro Gln Arg Lys Arg Pro Ala
   1 5 10
<210> 4
   <211> 150
   <212> PRT
   <213> Artificial Sequence
<220>
   <223> signal peptide
<400> 4
   Met Thr Gly Ala Ser Arg Arg Ser Ala Arg Gly Arg Ile Lys Ser Ser
   1               5                   10                  15
   Ser Leu Ser Pro Gly Ser Asp Glu Gly Ser Ala Tyr Pro Pro Ser Ile
               20                  25                  30
   Arg Arg Gly Lys Gly Lys Glu Leu Val Ser Ile Gly Ala Phe Lys Thr
           35                  40                  45
   Asn Leu Lys Ile Leu Val Gly Leu Ile Ile Leu Gly Ile Ile Val Ile
       50                  55                  60
   Tyr Phe Val Ile Asn Arg Leu Val Arg His Gly Leu Leu Phe Asp Glu
   65                  70                  75                  80
   Ser Gln Lys Pro Arg Val Ile Thr Pro Phe Pro Ala Pro Lys Val Met
                   85                  90                  95
   Asp Leu Ser Met Phe Gln Gly Glu His Lys Glu Ser Leu Tyr Trp Gly
               100                 105                 110
   Thr Tyr Arg Pro His Val Tyr Phe Gly Val Arg Ala Arg Thr Pro Leu
           115                 120                 125
   Ser Leu Val Ala Gly Leu Met Trp Leu Gly Val Lys Asp Glu Met Tyr
       130                 135                 140
   Val Met Arg His Phe Cys
   145                 150
<210> 5
   <211> 49
   <212> PRT
   <213> Artificial Sequence
<220>
   <223> signal peptide
<400> 5
   Met Ala Arg Gly Ser Arg Ser Val Gly Ser Ser Ser Ser Lys Trp Arg
   1               5                   10                  15
   Tyr Cys Asn Pro Ser Tyr Tyr Leu Lys Arg Pro Lys Arg Leu Ala Leu
               20                  25                  30
   Leu Phe Ile Val Phe Val Cys Val Ser Phe Val Phe Trp Asp Arg Gln
           35                  40                  45
   Thr
<210> 6
   <211> 99
   <212> PRT
   <213> Artificial Sequence
<220>
   <223> signal peptide
<400> 6
   Met Ala Arg Gly Ser Arg Ser Val Gly Ser Ser Ser Ser Lys Trp Arg
   1               5                   10                  15
   Tyr Cys Asn Pro Ser Tyr Tyr Leu Lys Arg Pro Lys Arg Leu Ala Leu
               20                  25                  30
   Leu Phe Ile Val Phe Val Cys Val Ser Phe Val Phe Trp Asp Arg Gln
           35                  40                  45
   Thr Leu Val Arg Glu His Gln Val Glu Ile Ser Glu Leu Gln Lys Glu
       50                  55                  60
   Val Thr Asp Leu Lys Asn Leu Val Asp Asp Leu Asn Asn Lys Gln Gly
   65                  70                  75                  80
   Gly Thr Ser Gly Lys Thr Asp Leu Gly Arg Lys Ala Thr Lys Ser Ser
                   85                  90                  95
   Lys Asp Val
<210> 7
   <211> 22
   <212> PRT
   <213> Artificial Sequence
<220>
   <223> signal peptide
<400> 7
   Met Ala Ala Ala Leu Ala Leu Leu Phe Ile Val Phe Val Cys Val Ser
   1               5                   10                  15
   Phe Val Phe Trp Asp Arg
               20
<210> 8
   <211> 68
   <212> PRT
   <213> Artificial Sequence
<220>
   <223> signal peptide
<400> 8
   Met Gly Val Phe Ser Asn Leu Arg Gly Pro Arg Ala Gly Ala Thr His
   1               5                   10                  15
   Asp Glu Phe Pro Ala Thr Asn Gly Ser Pro Ser Ser Ser Ser Ser Pro
               20                  25                  30
   Ser Ser Ser Ile Lys Arg Lys Leu Ser Asn Leu Leu Pro Leu Cys Val
           35                  40                  45
   Ala Leu Val Val Ile Ala Glu Ile Gly Phe Leu Gly Arg Leu Asp Lys
       50                  55                  60
   Val Ala Thr Ser
   65
<210> 9
   <211> 38
   <212> PRT
   <213> Artificial Sequence
<220>
   <223> signal peptide
<400> 9
   Met Arg Gly Tyr Lys Phe Cys Cys Asp Phe Arg Tyr Leu Leu Ile Leu
   1               5                   10                  15
   Ala Ala Val Ala Phe Ile Tyr Ile Gln Met Arg Leu Phe Ala Thr Gln
               20                  25                  30
   Ser Glu Tyr Ala Asp Arg
           35
<210> 10
   <211> 68
   <212> PRT
   <213> Artificial Sequence
<220>
   <223> signal peptide
<400> 10
   Met Gly Val Phe Ser Asn Leu Arg Gly Pro Lys Ile Gly Leu Thr His
   1 5 10 15
   Glu Glu Leu Pro Val Val Ala Asn Gly Ser Thr Ser Ser Ser Ser Ser
   20 25 30
   Pro Ser Ser Phe Lys Arg Lys Val Ser Thr Phe Leu Pro Ile Cys Val
   35 40 45
   Ala Leu Val Val Ile Ile Glu Ile Gly Phe Leu Cys Arg Leu Asp Asn
   50 55 60
   Ala Ser Thr Ser
   65
<210> 11
   <211> 41
   <212> PRT
   <213> Artificial Sequence
<220>
   <223> signal peptide
<400> 11
   Met Leu Val Met Pro Gln Pro Pro Lys Pro Phe Asn Thr Ile Thr Ile
   1 5 10 15
   Thr Ile Met Ile Ala Phe Thr Phe Phe Leu Leu Phe Leu Thr Gly Phe
   20 25 30
   Leu Gln Phe Pro Ser Ile Ser Pro Ser
   35 40
<210> 12
   <211> 106
   <212> PRT
   <213> Artificial Sequence
<220>
   <223> signal peptide
<400> 12
   Met Ala Arg Gly Ser Arg Ser Val Gly Ser Ser Ser Ser Lys Trp Arg
   1               5                   10                  15
   Tyr Cys Asn Pro Ser Tyr Tyr Leu Lys Arg Pro Lys Arg Leu Ala Leu
               20                  25                  30
   Leu Phe Ile Val Phe Val Cys Val Ser Phe Val Phe Trp Cys Val Ser
           35                  40                  45
   Phe Val Phe Trp Asp Arg Gln Thr Leu Val Arg Glu His Gln Val Glu
       50                  55                  60
   Ile Ser Glu Leu Gln Lys Glu Val Thr Asp Leu Lys Asn Leu Val Asp
   65                  70                  75                  80
   Asp Leu Asn Asn Lys Gln Gly Gly Thr Ser Gly Lys Thr Asp Leu Gly
                   85                  90                  95
   Arg Lys Ala Thr Lys Ser Ser Lys Asp Val
               100                 105
<210> 13
   <211> 21
   <212> PRT
   <213> Artificial Sequence
<220>
   <223> signal peptide
<220>
   <221> misc_feature
   <222> (1)..(3)
   <223> Xaa can be any naturally occurring amino acid
<400> 13
   Xaa Xaa Xaa Leu Ala Leu Leu Phe Ile Val Phe Val Cys Val Ser Phe
   1               5                   10                  15
   Val Phe Trp Asp Arg
               20
<210> 14
   <211> 25
   <212> PRT
   <213> Artificial Sequence
<220>
   <223> signal peptide
<220>
   <221> misc_feature
   <222> (1)..(2)
   <223> Xaa can be any naturally occurring amino acid
<400> 14
   Xaa Xaa Arg Tyr Leu Leu Ile Leu Ala Ala Val Ala Phe Ile Tyr Ile
   1               5                   10                  15
   Gln Met Arg Leu Phe Ala Thr Gln Ser
               20                  25
<210> 15
   <211> 24
   <212> PRT
   <213> Artificial Sequence
<220>
   <223> signal peptide
<220>
   <221> misc_feature
   <222> (1)..(3)
   <223> Xaa can be any naturally occurring amino acid
<220>
   <221> misc_feature
   <222> (22) .. (24)
   <223> Xaa can be any naturally occurring amino acid
<400> 15
   Xaa Xaa Xaa Leu Gly Ile Leu Phe Ala Val Thr Leu Ser Ile Val Leu
   1               5                   10                  15
   Met Leu Val Ser Val Xaa Xaa Xaa
               20
<210> 16
   <211> 23
   <212> PRT
   <213> Artificial Sequence
<220>
   <223> signal peptide
<220>
   <221> misc_feature
   <222> (1)..(2)
   <223> Xaa can be any naturally occurring amino acid
<400> 16
   Xaa Xaa Lys Ile Phe Leu Tyr Met Leu Leu Leu Asn Ser Leu Phe Leu
   1               5                   10                  15
   Ile Ile Tyr Phe Val Phe His
               20
<210> 17
   <211> 26
   <212> PRT
   <213> Artificial Sequence
<220>
   <223> signal petide
<220>
   <221> misc_feature
   <222> (1)..(3)
   <223> Xaa can be any naturally occurring amino acid
<400> 17
   Xaa Xaa Xaa Arg Lys Leu Ser Asn Leu Leu Pro Leu Cys Val Ala Leu
   1               5                   10                  15
   Val Val Ile Ala Glu Ile Gly Phe Leu Gly
               20                  25
<210> 18
   <211> 26
   <212> PRT
   <213> Artificial Sequence
<220>
   <223> signal peptide
<220>
   <221> misc_feature
   <222> (1)..(3)
   <223> Xaa can be any naturally occurring amino acid
<400> 18
   Xaa Xaa Xaa Arg Lys Val Ser Thr Phe Leu Pro Ile Cys Val Ala Leu
   1               5                   10                  15
   Val Val Ile Ile Glu Ile Gly Phe Leu Cys
               20                  25
<210> 19
   <211> 29
   <212> PRT
   <213> Artificial Sequence
<220>
   <223> signal peptide
<220>
   <221> misc_feature
   <222> (1)..(2)
   <223> Xaa can be any naturally occurring amino acid
<220>
   <221> misc_feature
   <222> (28) .. (29)
   <223> Xaa can be any naturally occurring amino acid
<400> 19
   Xaa Xaa Phe Asn Thr Ile Thr Ile Thr Ile Met Ile Ala Phe Thr Phe
   1               5                   10                  15
   Phe Leu Leu Phe Leu Thr Gly Phe Leu Gln Phe Xaa Xaa
                       20                  25
<210> 20
   <211> 29
   <212> PRT
   <213> Artificial Sequence
<220>
   <223> signal peptide
<220>
   <221> misc_feature
   <222> (1)..(2)
   <223> Xaa can be any naturally occurring amino acid
<400> 20
   Xaa Xaa Lys Arg Leu Ala Leu Leu Phe Ile Val Phe Val Cys Val Ser
   1               5                   10                  15
   Phe Val Phe Trp Cys Val Ser Phe Val Phe Trp Asp Arg
               20                  25
<210> 21
   <211> 12295
   <212> DNA
   <213> Artificial Sequence
<220>
   <223> plasmid pAG01
<400> 21
   tgagcgtcgc aaaggcgctc ggtcttgcct tgctcgtcgg tgatgtactt caccagctcc        60
   gcgaagtcgc tcttcttgat ggagcgcatg gggacgtgct tggcaatcac gcgcaccccc       120
   cggccgtttt agcggctaaa aaagtcatgg ctctgccctc gggcggacca cgcccatcat       180
   gaccttgcca agctcgtcct gcttctcttc gatcttcgcc agcagggcga ggatcgtggc       240
   atcaccgaac cgcgccgtgc gcgggtcgtc ggtgagccag agtttcagca ggccgcccag       300
   gcggcccagg tcgccattga tgcgggccag ctcgcggacg tgctcatagt ccacgacgcc       360
   cgtgattttg tagccctggc cgacggccag caggtaggcc gacaggctca tgccggccgc       420
   cgccgccttt tcctcaatcg ctcttcgttc gtctggaagg cagtacacct tgataggtgg       480
   gctgcccttc ctggttggct tggtttcatc agccatccgc ttgccctcat ctgttacgcc       540
   ggcggtagcc ggccagcctc gcagagcagg attcccgttg agcaccgcca ggtgcgaata       600
   agggacagtg aagaaggaac acccgctcgc gggtgggcct acttcaccta tcctgcccgg       660
   ctgacgccgt tggatacacc aaggaaagtc tacacgaacc ctttggcaaa atcctgtata       720
   tcgtgcgaaa aaggatggat ataccgaaaa aatcgctata atgaccccga agcagggtta       780
   tgcagcggaa aagcgccacg cttcccgaag ggagaaaggc ggacaggtat ccggtaagcg       840
   gcagggtcgg aacaggagag cgcacgaggg agcttccagg gggaaacgcc tggtatcttt 900
   atagtcctgt cgggtttcgc cacctctgac ttgagcgtcg atttttgtga tgctcgtcag 960
   gggggcggag cctatggaaa aacgccagca acgcggcctt tttacggttc ctggcctttt 1020
   gctggccttt tgctcacatg ttctttcctg cgttatcccc tgattctgtg gataaccgta 1080
   ttaccgcctt tgagtgagct gataccgctc gccgcagccg aacgaccgag cgcagcgagt 1140
   cagtgagcga ggaagcggaa gagcgccaga aggccgccag agaggccgag cgcggccgtg 1200
   aggcttggac gctagggcag ggcatgaaaa agcccgtagc gggctgctac gggcgtctga 1260
   cgcggtggaa agggggaggg gatgttgtct acatggctct gctgtagtga gtgggttgcg 1320
   ctccggcagc ggtcctgatc aatcgtcacc ctttctcggt ccttcaacgt tcctgacaac 1380
   gagcctcctt ttcgccaatc catcgacaat caccgcgagt ccctgctcga acgctgcgtc 1440
   cggaccggct tcgtcgaagg cgtctatcgc ggcccgcaac agcggcgaga gcggagcctg 1500
   ttcaacggtg ccgccgcgct cgccggcatc gctgtcgccg gcctgctcct caagcacggc 1560
   cccaacagtg aagtagctga ttgtcatcag cgcattgacg gcgtccccgg ccgaaaaacc 1620
   cgcctcgcag aggaagcgaa gctgcgcgtc ggccgtttcc atctgcggtg cgcccggtcg 1680
   cgtgccggca tggatgcgcg cgccatcgcg gtaggcgagc agcgcctgcc tgaagctgcg 1740
   ggcattcccg atcagaaatg agcgccagtc gtcgtcggct ctcggcaccg aatgcgtatg 1800
   attctccgcc agcatggctt cggccagtgc gtcgagcagc gcccgcttgt tcctgaagtg 1860
   ccagtaaagc gccggctgct gaacccccaa ccgttccgcc agtttgcgtg tcgtcagacc 1920
   gtctacgccg acctcgttca acaggtccag ggcggcacgg atcactgtat tcggctgcaa 1980
   ctttgtcatg cttgacactt tatcactgat aaacataata tgtccaccaa cttatcagtg 2040
   ataaagaatc cgcgcgttca atcggaccag cggaggctgg tccggaggcc agacgtgaaa 2100
   cccaacatac ccctgatcgt aattctgagc actgtcgcgc tcgacgctgt cggcatcggc 2160
   ctgattatgc cggtgctgcc gggcctcctg cgcgatctgg ttcactcgaa cgacgtcacc 2220
   gcccactatg gcattctgct ggcgctgtat gcgttggtgc aatttgcctg cgcacctgtg 2280
   ctgggcgcgc tgtcggatcg tttcgggcgg cggccaatct tgctcgtctc gctggccggc 2340
   gccagatctg gggaaccctg tggttggcat gcacatacaa atggacgaac ggataaacct 2400
   tttcacgccc ttttaaatat ccgattattc taataaacgc tcttttctct taggtttacc 2460
   cgccaatata tcctgtcaaa cactgatagt ttaaactgaa ggcgggaaac gacaatctga 2520
   tcatgagcgg agaattaagg gagtcacgtt atgacccccg ccgatgacgc gggacaagcc 2580
   gttttacgtt tggaactgac agaaccgcaa cgttgaagga gccactcagc cgcgggtttc 2640
   tggagtttaa tgagctaagc acatacgtca gaaaccatta ttgcgcgttc aaaagtcgcc 2700
   taaggtcact atcagctagc aaatatttct tgtcaaaaat gctccactga cgttccataa 2760
   attcccctcg gtatccaatt agagtctcat attcactctc aatccaaata atctgcaccg 2820
   gatctggatc gtttcgcgtg cacaaaaatg gaacgagcta tacaaggaaa cgacgctagg 2880
   gaacaagcta acagtgaacg ttgggatgga ggatcaggag gcaccacttc tcccttcaaa 2940
   cttcctgacg aaagtccgag ttggactgag tggcggctac ataacgatga gacgaactcg 3000
   aatcaagata atccccttgg tttcaaggaa agctggggtt tcgggaaagt tgtatttaag 3060
   agatatctca gatacgacag gacggaagcc tcactgcaca gagtccttgg atcttggacg 3120
   ggagattcgg ttaactatgc agcatctcga tttttcggtt tcgaccagat cggatgtacc 3180
   tatagtattc ggtttcgagg agttagtatc accgtttctg gagggtcgcg aactcttcag 3240
   catctctgtg agatggcaat tcggtctaag caagaactgc tacagcttgc cccaatcgaa 3300
   gtggaaagta atgtatcaag aggatgccct gaaggtactg agaccttcga aaaagaaagc 3360
   gagtaagcgg gactctgggg ttcgaaatga ccgaccatat cttgctgcgt tcggatattt 3420
   tcgtggagtt cccgccacag acccggatga tcccctaatt cgggggatct ggattttagt 3480
   actggatttt ggttttagga attagaaatt ttattgatag aagtatttta caaatacaaa 3540
   tacatactaa gggtttctta tatgctcaac acatgagcga aaccctatag gaaccctaat 3600
   tcccttatct gggaactact cacacattat tatggagaaa ctcgagcttg tcgatcgacc 3660
   ctcctgtcaa tgctggcggc ggctctggtg gtggttctgg tggcggctct gagggtggtg 3720
   gctctgaggg tggcggttct gagggtggcg gctctgaggg aggcggttcc ggtggtggct 3780
   ctggttccgg tgattttgat tatgaaaaga tggcaaacgc taataagggg gctatgaccg 3840
   aaaatgccga tgaaaacgcg ctacagtctg acgctaaagg caaacttgat tctgtcgcta 3900
   ctgattacgg tgctgctatc gatggtttca ttggtgacgt ttccggcctt gctaatggta 3960
   atggtgctac tggtgatttt gctggctcta attcccaaat ggctcaagtc ggtgacggtg 4020
   ataattcacc tttaatgaat aatttccgtc aatatttacc ttccctccct caatcggttg 4080
   aatgtcgccc ttttgtcttt ggcccaatac gcaaaccgcc tctccccgcg cgttggccga 4140
   ttcattaatg cagctggcac gacaggtttc ccgactggaa agcgggcagt gagcgcaacg 4200
   caattaatgt gagttagctc actcattagg caccccaggc tttacacttt atgcttccgg 4260
   ctcgtatgtt gtgtggaatt gtgagcggat aacaatttca cacaggaaac agctatgacc 4320
   atgattacgc caagcttgca tgcctgcagg tccccagatt agccttttca atttcagaaa 4380
   gaatgctaac ccacagatgg ttagagaggc ttacgcagca ggtctcatca agacgatcta 4440
   cccgagcaat aatctccagg aaatcaaata ccttcccaag aaggttaaag atgcagtcaa 4500
   aagattcagg actaactgca tcaagaacac agagaaagat atatttctca agatcagaag 4560
   tactattcca gtatggacga ttcaaggctt gcttcacaaa ccaaggcaag taatagagat 4620
   tggagtctct aaaaaggtag ttcccactga atcaaaggcc atggagtcaa agattcaaat 4680
   agaggaccta acagaactcg ccgtaaagac tggcgaacag ttcatacaga gtctcttacg 4740
   actcaatgac aagaagaaaa tcttcgtcaa catggtggag cacgacacac ttgtctactc 4800
   caaaaatatc aaagatacag tctcagaaga ccaaagggca attgagactt ttcaacaaag 4860
   ggtaatatcc ggaaacctcc tcggattcca ttgcccagct atctgtcact ttattgtgaa 4920
   gatagtggaa aaggaaggtg gctcctacaa atgccatcat tgcgataaag gaaaggccat 4980
   cgttgaagat gcctctgccg acagtggtcc caaagatgga cccccaccca cgaggagcat 5040
   cgtggaaaaa gaagacgttc caaccacgtc ttcaaagcaa gtggattgat gtgatatctc 5100
   cactgacgta agggatgacg cacaatccca ctatccttcg caagaccctt cctctatata 5160
   aggaagttca tttcatttgg agagaacacg ggggactcta gaggtacccg ggcccgcgga 5220
   tccgcggccg cactagtcga caggcctgag ctcgaatttc cccgatcgtt caaacatttg 5280
   gcaataaagt ttcttaagat tgaatcctgt tgccggtctt gcgatgatta tcatataatt 5340
   tctgttgaat tacgttaagc atgtaataat taacatgtaa tgcatgacgt tatttatgag 5400
   atgggttttt atgattagag tcccgcaatt atacatttaa tacgcgatag aaaacaaaat 5460
   atagcgcgca aactaggata aattatcgcg cgcggtgtca tctatgttac tagatcggga 5520
   attcactggc cgtcgtttta caacgtcgtg actgggaaaa ccctggcgtt acccaactta 5580
   atcgccttgc agcacatccc cctttcgcca gctggcgtaa tagcgaagag gcccgcaccg 5640
   atcgcccttc ccaacagttg cgcagcctga atggcgcccg ctcctttcgc tttcttccct 5700
   tcctttctcg ccacgttcgc cggctttccc cgtcaagctc taaatcgggg gctcccttta 5760
   gggttccgat ttagtgcttt acggcacctc gaccccaaaa aacttgattt gggtgatggt 5820
   tcacgtagtg ggccatcgcc ctgatagacg gtttttcgcc ctttgacgtt ggagtccacg 5880
   ttctttaata gtggactctt gttccaaact ggaacaacac tcaaccctat ctcgggctat 5940
   tcttttgatt tataagggat tttgccgatt tcggaaccac catcaaacag gattttcgcc 6000
   tgctggggca aaccagcgtg gaccgcttgc tgcaactctc tcagggccag gcggtgaagg 6060
   gcaatcagct gttgcccgtc tcactggtga aaagaaaaac caccccagta cattaaaaac 6120
   gtccgcaatg tgttattaag ttgtctaagc gtcaatttgt ttacaccaca atatatcctg 6180
   ccaccagcca gccaacagct ccccgaccgg cagctcggca caaaatcacc actcgataca 6240
   ggcagcccat cagtccggga cggcgtcagc gggagagccg ttgtaaggcg gcagactttg 6300
   ctcatgttac cgatgctatt cggaagaacg gcaactaagc tgccgggttt gaaacacgga 6360
   tgatctcgcg gagggtagca tgttgattgt aacgatgaca gagcgttgct gcctgtgatc 6420
   aaatatcatc tccctcgcag agatccgaat tatcagcctt cttattcatt tctcgcttaa 6480
   ccgtgacagg ctgtcgatct tgagaactat gccgacataa taggaaatcg ctggataaag 6540
   ccgctgagga agctgagtgg cgctatttct ttagaagtga acgttgacga tatcaactcc 6600
   cctatccatt gctcaccgaa tggtacaggt cggggacccg aagttccgac tgtcggcctg 6660
   atgcatcccc ggctgatcga ccccagatct ggggctgaga aagcccagta aggaaacaac 6720
   tgtaggttcg agtcgcgaga tcccccggaa ccaaaggaag taggttaaac ccgctccgat 6780
   caggccgagc cacgccaggc cgagaacatt ggttcctgta ggcatcggga ttggcggatc 6840
   aaacactaaa gctactggaa cgagcagaag tcctccggcc gccagttgcc aggcggtaaa 6900
   ggtgagcaga ggcacgggag gttgccactt gcgggtcagc acggttccga acgccatgga 6960
   aaccgccccc gccaggcccg ctgcgacgcc gacaggatct agcgctgcgt ttggtgtcaa 7020
   caccaacagc gccacgcccg cagttccgca aatagccccc aggaccgcca tcaatcgtat 7080
   cgggctacct agcagagcgg cagagatgaa cacgaccatc agcggctgca cagcgcctac 7140
   cgtcgccgcg accccgcccg gcaggcggta gaccgaaata aacaacaagc tccagaatag 7200
   cgaaatatta agtgcgccga ggatgaagat gcgcatccac cagattcccg ttggaatctg 7260
   tcggacgatc atcacgagca ataaacccgc cggcaacgcc cgcagcagca taccggcgac 7320
   ccctcggcct cgctgttcgg gctccacgaa aacgccggac agatgcgcct tgtgagcgtc 7380
   cttggggccg tcctcctgtt tgaagaccga cagcccaatg atctcgccgt cgatgtaggc 7440
   gccgaatgcc acggcatctc gcaaccgttc agcgaacgcc tccatgggct ttttctcctc 7500
   gtgctcgtaa acggacccga acatctctgg agctttcttc agggccgaca atcggatctc 7560
   gcggaaatcc tgcacgtcgg ccgctccaag ccgtcgaatc tgagccttaa tcacaattgt 7620
   caattttaat cctctgttta tcggcagttc gtagagcgcg ccgtgcgtcc cgagcgatac 7680
   tgagcgaagc aagtgcgtcg agcagtgccc gcttgttcct gaaatgccag taaagcgctg 7740
   gctgctgaac ccccagccgg aactgacccc acaaggccct agcgtttgca atgcaccagg 7800
   tcatcattga cccaggcgtg ttccaccagg ccgctgcctc gcaactcttc gcaggcttcg 7860
   ccgacctgct cgcgccactt cttcacgcgg gtggaatccg atccgcacat gaggcggaag 7920
   gtttccagct tgagcgggta cggctcccgg tgcgagctga aatagtcgaa catccgtcgg 7980
   gccgtcggcg acagcttgcg gtacttctcc catatgaatt tcgtgtagtg gtcgccagca 8040
   aacagcacga cgatttcctc gtcgatcagg acctggcaac gggacgtttt cttgccacgg 8100
   tccaggacgc ggaagcggtg cagcagcgac accgattcca ggtgcccaac gcggtcggac 8160
   gtgaagccca tcgccgtcgc ctgtaggcgc gacaggcatt cctcggcctt cgtgtaatac 8220
   cggccattga tcgaccagcc caggtcctgg caaagctcgt agaacgtgaa ggtgatcggc 8280
   tcgccgatag gggtgcgctt cgcgtactcc aacacctgct gccacaccag ttcgtcatcg 8340
   tcggcccgca gctcgacgcc ggtgtaggtg atcttcacgt ccttgttgac gtggaaaatg 8400
   accttgtttt gcagcgcctc gcgcgggatt ttcttgttgc gcgtggtgaa cagggcagag 8460
   cgggccgtgt cgtttggcat cgctcgcatc gtgtccggcc acggcgcaat atcgaacaag 8520
   gaaagctgca tttccttgat ctgctgcttc gtgtgtttca gcaacgcggc ctgcttggcc 8580
   tcgctgacct gttttgccag gtcctcgccg gcggtttttc gcttcttggt cgtcatagtt 8640
   cctcgcgtgt cgatggtcat cgacttcgcc aaacctgccg cctcctgttc gagacgacgc 8700
   gaacgctcca cggcggccga tggcgcgggc agggcagggg gagccagttg cacgctgtcg 8760
   cgctcgatct tggccgtagc ttgctggacc atcgagccga cggactggaa ggtttcgcgg 8820
   ggcgcacgca tgacggtgcg gcttgcgatg gtttcggcat cctcggcgga aaaccccgcg 8880
   tcgatcagtt cttgcctgta tgccttccgg tcaaacgtcc gattcattca ccctccttgc 8940
   gggattgccc cgactcacgc cggggcaatg tgcccttatt cctgatttga cccgcctggt 9000
   gccttggtgt ccagataatc caccttatcg gcaatgaagt cggtcccgta gaccgtctgg 9060
   ccgtccttct cgtacttggt attccgaatc ttgccctgca cgaataccag cgaccccttg 9120
   cccaaatact tgccgtgggc ctcggcctga gagccaaaac acttgatgcg gaagaagtcg 9180
   gtgcgctcct gcttgtcgcc ggcatcgttg cgccacatct aggtactaaa acaattcatc 9240
   cagtaaaata taatatttta ttttctccca atcaggcttg atccccagta agtcaaaaaa 9300
   tagctcgaca tactgttctt ccccgatatc ctccctgatc gaccggacgc agaaggcaat 9360
   gtcataccac ttgtccgccc tgccgcttct cccaagatca ataaagccac ttactttgcc 9420
   atctttcaca aagatgttgc tgtctcccag gtcgccgtgg gaaaagacaa gttcctcttc 9480
   gggcttttcc gtctttaaaa aatcatacag ctcgcgcgga tctttaaatg gagtgtcttc 9540
   ttcccagttt tcgcaatcca catcggccag atcgttattc agtaagtaat ccaattcggc 9600
   taagcggctg tctaagctat tcgtataggg acaatccgat atgtcgatgg agtgaaagag 9660
   cctgatgcac tccgcataca gctcgataat cttttcaggg ctttgttcat cttcatactc 9720
   ttccgagcaa aggacgccat cggcctcact catgagcaga ttgctccagc catcatgccg 9780
   ttcaaagtgc aggacctttg gaacaggcag ctttccttcc agccatagca tcatgtcctt 9840
   ttcccgttcc acatcatagg tggtcccttt ataccggctg tccgtcattt ttaaatatag 9900
   gttttcattt tctcccacca gcttatatac cttagcagga gacattcctt ccgtatcttt 9960
   tacgcagcgg tatttttcga tcagtttttt caattccggt gatattctca ttttagccat 10020
   ttattatttc cttcctcttt tctacagtat ttaaagatac cccaagaagc taattataac 10080
   aagacgaact ccaattcact gttccttgca ttctaaaacc ttaaatacca gaaaacagct 10140
   ttttcaaagt tgttttcaaa gttggcgtat aacatagtat cgacggagcc gattttgaaa 10200
   ccacaattat gggtgatgct gccaacttac tgatttagtg tatgatggtg tttttgaggt 10260
   gctccagtgg cttctgtgtc tatcagctgt ccctcctgtt cagctactga cggggtggtg 10320
   cgtaacggca aaagcaccgc cggacatcag cgctatctct gctctcactg ccgtaaaaca 10380
   tggcaactgc agttcactta caccgcttct caacccggta cgcaccagaa aatcattgat 10440
   atggccatga atggcgttgg atgccgggca acagcccgca ttatgggcgt tggcctcaac 10500
   acgattttac gtcacttaaa aaactcaggc cgcagtcggt aacctcgcgc atacagccgg 10560
   gcagtgacgt catcgtctgc gcggaaatgg acgaacagtg gggctatgtc ggggctaaat 10620
   cgcgccagcg ctggctgttt tacgcgtatg acagtctccg gaagacggtt gttgcgcacg 10680
   tattcggtga acgcactatg gcgacgctgg ggcgtcttat gagcctgctg tcaccctttg 10740
   acgtggtgat atggatgacg gatggctggc cgctgtatga atcccgcctg aagggaaagc 10800
   tgcacgtaat cagcaagcga tatacgcagc gaattgagcg gcataacctg aatctgaggc 10860
   agcacctggc acggctggga cggaagtcgc tgtcgttctc aaaatcggtg gagctgcatg 10920
   acaaagtcat cgggcattat ctgaacataa aacactatca ataagttgga gtcattaccc 10980
   aattatgata gaatttacaa gctataaggt tattgtcctg ggtttcaagc attagtccat 11040
   gcaagttttt atgctttgcc cattctatag atatattgat aagcgcgctg cctatgcctt 11100
   gccccctgaa atccttacat acggcgatat cttctatata aaagatatat tatcttatca 11160
   gtattgtcaa tatattcaag gcaatctgcc tcctcatcct cttcatcctc ttcgtcttgg 11220
   tagcttttta aatatggcgc ttcatagagt aattctgtaa aggtccaatt ctcgttttca 11280
   tacctcggta taatcttacc tatcacctca aatggttcgc tgggtttatc gcacccccga 11340
   acacgagcac ggcacccgcg accactatgc caagaatgcc caaggtaaaa attgccggcc 11400
   ccgccatgaa gtccgtgaat gccccgacgg ccgaagtgaa gggcaggccg ccacccaggc 11460
   cgccgccctc actgcccggc acctggtcgc tgaatgtcga tgccagcacc tgcggcacgt 11520
   caatgcttcc gggcgtcgcg ctcgggctga tcgcccatcc cgttactgcc ccgatcccgg 11580
   caatggcaag gactgccagc gctgccattt ttggggtgag gccgttcgcg gccgaggggc 11640
   gcagcccctg gggggatggg aggcccgcgt tagcgggccg ggagggttcg agaagggggg 11700
   gcacccccct tcggcgtgcg cggtcacgcg cacagggcgc agccctggtt aaaaacaagg 11760
   tttataaata ttggtttaaa agcaggttaa aagacaggtt agcggtggcc gaaaaacggg 11820
   cggaaaccct tgcaaatgct ggattttctg cctgtggaca gcccctcaaa tgtcaatagg 11880
   tgcgcccctc atctgtcagc actctgcccc tcaagtgtca aggatcgcgc ccctcatctg 11940
   tcagtagtcg cgcccctcaa gtgtcaatac cgcagggcac ttatccccag gcttgtccac 12000
   atcatctgtg ggaaactcgc gtaaaatcag gcgttttcgc cgatttgcga ggctggccag 12060
   ctccacgtcg ccggccgaaa tcgagcctgc ccctcatctg tcaacgccgc gccgggtgag 12120
   tcggcccctc aagtgtcaac gtccgcccct catctgtcag tgagggccaa gttttccgcg 12180
   aggtatccac aacgccggcg gccgcggtgt ctcgcacacg gcttcgacgg cgtttctggc    12240
   gcgtttgcag ggccatagac ggccgccagc ccagcggcga gggcaaccag cccgg         12295

## Description

La présente invention se rapporte à une méthode de production d'allergènes recombinants de haute qualité.

L'utilisation d'allergènes recombinants permet une plus grande spécificité et une meilleure efficacité des tests diagnostic et du traitement des allergies.

De nombreux allergènes ont déjà été produits sous forme recombinante. Ils sont utilisés aujourd'hui pour le diagnostic *in vitro* des allergies. Toutefois le système d'expression utilisé, généralement *E. coli*, ne permet, le plus souvent, d'obtenir que des copies très approximatives des allergènes naturels, en raison de l'incapacité de cette bactérie à réaliser les modifications post-traductionnelles nécessaires pour un repliement correct de protéines d'eucaryotes. Ceci nuit souvent à la fiabilité et à la sensibilité des tests diagnostiques réalisés en utilisant ces molécules, puisque certains épitopes susceptibles de réagir avec les immunoglobulines E (IgE) de patients ne sont pas présents sur les allergènes recombinants produits chez *E.coli.*

Des systèmes d'expression eucaryotes ont également été utilisés pour la production d'allergènes recombinants. Ce sont le plus souvent des levures, et dans ce cas l'hyperglycosylation, spécifique de ces organismes, ne permet pas non plus la production d'allergènes recombinants conformes à leurs homologues naturels.

Les plantes sont les seuls hôtes eucaryotes permettant la production d'allergènes complexes sous forme recombinante, pour des coûts de production et d'une qualité compatibles avec leur utilisation pour un traitement personnalisé des allergies, intégrant les tests diagnostic et la thérapie.

En revanche, les systèmes végétaux d'expression utilisés jusqu'ici pour la production d'allergènes recombinants font généralement appel à la transgénèse végétale avec ses principales limites, bien connues, à savoir :
- des temps longs de passage du gène à la protéine ; ce qui signifie un effort de développement sur plusieurs années, et
- des rendements faibles, de l'ordre de 0,1% à 1% des protéines solubles ; ce qui signifie le traitement d'une grande biomasse de matériel végétal pour une production à grande échelle.

Les progrès récents réalisés grâce à l'expression transitoire ont permis de dépasser ces limites, d'une part en réduisant considérablement les délais de passage du gène à la protéine, ce qui permet un effort de développement beaucoup plus rapide, et d'autre part en augmentant les rendements de production au minimum d'un facteur 10, ce qui minimise les investissements pour l'extraction et la purification de la protéine d'intérêt.

Une telle technique d'expression transitoire chez les végétaux est utilisée aujourd'hui à grande échelle pour la production de vaccins par certaines sociétés, qui développent actuellement de vastes unités de production aux Etats-Unis.

Cependant, malgré ces efforts, il existe toujours un besoin de disposer d'une méthode efficace et reproductible de production d'allergènes recombinants, qui permet l'obtention d'allergènes recombinants ayant une composition et une conformation semblables à celles de leurs homologues naturels. Par ailleurs, il existe un besoin pour une méthode ayant un bon rendement de production.

La présente invention permet d'obtenir des protéines recombinantes complexes, en particulier des allergènes recombinants complexes, qui n'ont jamais été obtenus auparavant sous forme recombinante. Ces allergènes sont de plus des copies conformes de leurs homologues naturels.

La présente invention se rapporte donc à un procédé de production d'une protéine recombinante dans une plante de tabac *Nicotiana benthamiana*, comprenant les étapes suivantes :
a) la culture des plantes en aéroponie ou en hydroponie, de préférence sur flotteurs mobiles, et sous éclairage LEDs,
b) l'agroinfiltration des plantes obtenue à l'étape a), sous vide, par des agrobactéries comprenant un vecteur d'expression comprenant un fragment d'ADN codant pour la protéine recombinante, puis
c) la remise en culture des plantes après l'étape b), dans les mêmes conditions que pour l'étape a), puis
d) l'extraction et la purification de la protéine recombinante à partir des parties aériennes des plantes produites à l'étape c).

La présente demande décrit également une protéine recombinante susceptible d'être obtenue par le procédé selon l'invention.

La plante utilisable dans le procédé décrit ici est notamment une plante de tabac choisie parmi *Nicotiana benthamiana* et *Nicotiana tabacum* ou encore tout autre plante utilisable pour l'expression transitoire, comme une laitue (genre Lactuca) ou une plante d'épinard (Spinacia oleracea). Parmi les laitues, on peut citer la laitue Appia, Grosse Blonde Paresseuse, Lollo Rossa, Merveille de quatre saisons, feuille de chêne, ou red sails. La plante peut également être du genre Arabidopsis, ou un de ses mutants, en particulier des mutants de glycosylation d'Arabidopsis ; enfin, on peut utiliser des plantes knock out de tabac (en particulier de mutants de glycosylation). La plante utilisée selon l'invention est *N. benthamiana.*

La protéine recombinante produite par le procédé selon l'invention est un allergène recombinant, de préférence un allergène recombinant d'acarien.
Par « allergène », on entend toute protéine ou tout peptide capable de provoquer une réaction allergique chez un sujet préalablement sensibilisé lorsqu'il est à son contact, le plus souvent par contact avec la peau, inhalation ou ingestion. Un allergène est dit « majeur » quand un antigène purifié déclenche une allergie chez 50% ou plus des patients testés, et qu'il présente des IgE spécifiques, avec des tests cutanés immédiatement positifs, à une concentration très faible, chez au moins 70% des sujets ayant la maladie allergique en relation avec cet allergène.
Par « protéine », on entend une séquence comprenant au moins 50 acides aminés.
Par « peptide », on entend une séquence comprenant entre 1 et 49 acide aminés, de préférence entre 2 et 40 acides aminés.

De préférence, la protéine recombinante produite par le procédé selon l'invention est un allergène, un fragment d'allergène ou une protéine de fusion comprenant un allergène ou un fragment d'allergène.

De préférence, la protéine recombinante est choisie parmi les allergènes responsables d'allergies respiratoires issus d'acariens domestiques, tels que *Dermatophagoides farinae, Dermatophagoides pteronyssinus ou Euroglyphus manei*, les allergènes d'acariens de stockage tel que *Blomia tropicalis,* les allergènes d'acariens de type *Acarus siro* (autrefois nommé *Tyroglyphus farinae*), les allergènes de blattes, les allergènes de pollens d'arbres ou de graminées, les allergènes d'animaux (chat, chien cheval), les allergènes de moisissures, les allergènes responsables d'allergies de contact comme ceux du latex d'hévéa ou encore les allergènes responsables d'allergies alimentaires (lait, œuf, poisson, fruits).

Parmi les allergènes de *Dermatophagoides farinae*, on peut citer Der f 10, Der f11, Der f 13, Der f 14, Der f 15, Der f 16, Der f 17, Der f 18, Der f2, Der f 2.0101, Der f 2.0102, Der f 2.0103, Der f 2.0104, Der f 2.0105, Der f 2.0106, Der f 2.0107, Der f 2.0108, Der f 2.0109, Der f 2.0110, Der f 2.0111, Der f 2.0112, Der f 2.0113, Der f 2.0114, Der f 2.0115, Der f 2.0116, Der f2.0117, Der f20, Der f3, Der f4, Der f5, Der f6, Der f7, Der f8, Der f9 et Der fHSP70.

Parmi les allergènes de *Dermatophagoides pteronyssinus*, on peut citer Der p 10, Der p 11, Der p 14, Der p 15, Der p 18, Der p 2, Der p 2.0101, Der p 2.0102, Der p 2.0103, Der p 2.0104, Der p 2.0105, Der p 2.0106, Der p 2.0107, Der p 2.0108, Der p 2.0109, Der p 2.0110, Der p 2.0111, Der p 2.0112, Der p 2.0113, Der p 20, Der p 21, Der p 3, Der p 4, Der p 5, Der p 6, Der p 7, Der p 8, Der p 9.

Parmi les allergènes de *Blomia tropicalis*, on peut citer Blo t 1, Blo t 5 (qui présente une homologie de séquence de 40% avec Der p 5), Blo t 9, Blo t 10, Blo t 12 ou Blo t 21.

Tous ces allergènes sont bien connus, et leur séquence peut être retrouvée notamment dans des bases de données telles qu'Allergome (allergome.org), ou tout simplement dans UniProt.

Le procédé de production d'allergènes recombinants par expression transitoire selon l'invention, chez *N. benthamiana*, est très efficace, reproductible, et présente un bon rendement.

Le procédé de production de protéines recombinantes selon l'invention comprend une première étape de culture de la plante (étape a), en aéroponie ou en hydroponie, de préférence en culture sur flotteurs mobiles libres, et sous éclairage LED, afin de lui faire produire la protéine recombinante.
L'aéroponie correspond à la culture de la plante sur un support, généralement en matière plastique, associé à des vaporisations permanentes de solutions nutritives à base de sels minéraux.
L'hydroponie correspond à la culture hors sol de la plante. Cette dernière est cultivée sur substrat neutre et inerte, tel que du sable, des billes d'argile, des plaques de polystyrène ou de la laine de roche. Le substrat est régulièrement irrigué d'un courant de solution qui apporte des sels minéraux et des nutriments essentiels à la plante. Dans le procédé utilisé selon l'invention, les plantes de tabac, notamment *N. benthamiana*, sont cultivées de préférence en hydroponie sur des flotteurs libres, par exemple sur une plaque de polystyrène perforé. Ces flotteurs sont disposés dans des cuves contenant un milieu de culture aéré en permanence grâce à des diffuseurs d'air. Cette technique permet une standardisation des conditions de production des protéines recombinantes, couplée à l'absence totale de risque de contamination des milieux d'agroinfiltration lors de l'étape b), par des impuretés ou débris issus des substrats contenus dans les pots (dans le cas des cultures classiques). De plus, l'utilisation de ce système de culture permet d'atteindre des rendements très supérieurs, comme cela est montré dans les exemples.

Enfin, lors du scale-up, la manipulation pour l'agroinfiltration ou la récolte de lots de plantes fixées sur une plaque de polystyrène est évidemment plus facile que celle de plantes cultivées en pot et substrat.

Le procédé de production de protéines recombinantes selon l'invention comprend, suite à l'étape a), une étape b) d'agroinfiltration de la plante, notamment de la plante de tabac, sous vide, par des agrobactéries comprenant un fragment d'ADN codant pour la protéine recombinante.

Notamment, après cinq semaines de culture, de préférence en hydroponie sur des flotteurs mobiles libres, l'agroinfiltration des plantes de tabac est réalisée sous vide, par des agrobactéries comprenant vecteur d'expression comprenant un fragment d'ADN codant pour la protéine recombinante.

Cette étape b) d'agroinfiltration peut être mise en œuvre par tout moyen permettant de faire le vide. De préférence, dans le procédé utilisé selon l'invention, elle est réalisée sous vide par effet Venturi.

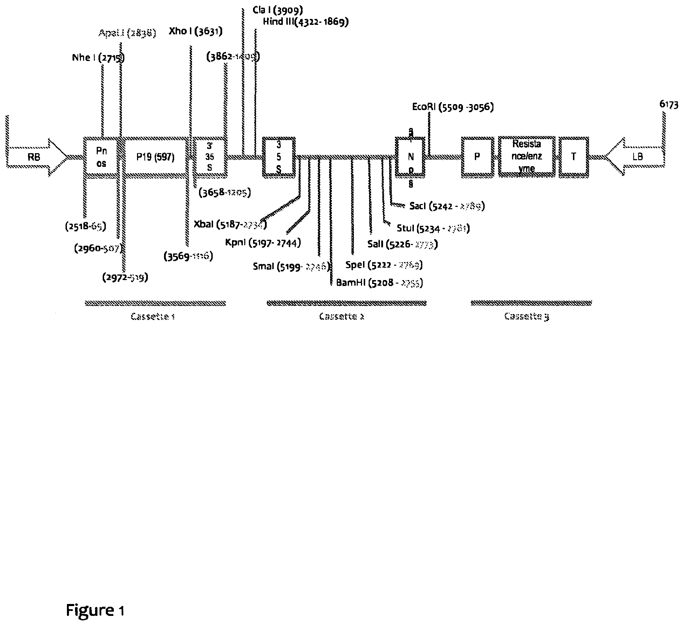

Le fragment d'ADN codant pour la protéine recombinante, utilisé dans l'étape a) et introduit dans les agrobactéries, peut être préparé par clonage. Ce fragment d'ADN peut comprendre la séquence codant pour la protéine recombinante, par exemple un allergène hétérologue, ladite séquence étant fusionnée à une séquence codant un peptide facilitant sa purification, comme par exemple une séquence « tag histidine » ou une séquence codant un peptide ou un polypeptide d'adressage intracellulaire. Un tel peptide ou polypeptide d'adressage intracellulaire peut notamment être choisi parmi les peptides dont les séquences sont présentées dans le tableau 1, i.e. parmi les peptides SEQ ID NO :1 à 20.
Le fragment d'ADN peut ensuite être intégré dans le vecteur d'expression pAG01 développé dans le cadre de l'invention (figure 1 et séquence SEQ ID NO : 21), puis les agrobactéries sont transformées à l'aide de ce vecteur d'expression. La présente demande décrit également un vecteur d'expression comprenant la séquence SEQ ID NO :21 et un insert, notamment localisé entre les limites droite et gauche de l'ADN de transfert (TDNA) (ceci est illustré par la figure 1, où la limite gauche est « LB », et la limite droite est « RB »), ledit insert comprenant au moins une séquence nucléique codant pour un peptide choisi parmi SEQ ID NO : 1 à 20, ladite séquence nucléique étant directement fusionnée à une seconde séquence nucléique codant la protéine d'intérêt. Ce vecteur correspond au vecteur pAG01 comprenant un insert, ledit insert comprenant une séquence nucléique de peptide (choisi parmi SEQ ID NO : 1 à 20) directement fusionnée à la séquence nucléique de l'allergène d'intérêt. De préférence, la protéine d'intérêt est un allergène tel que décrit ci-dessus.

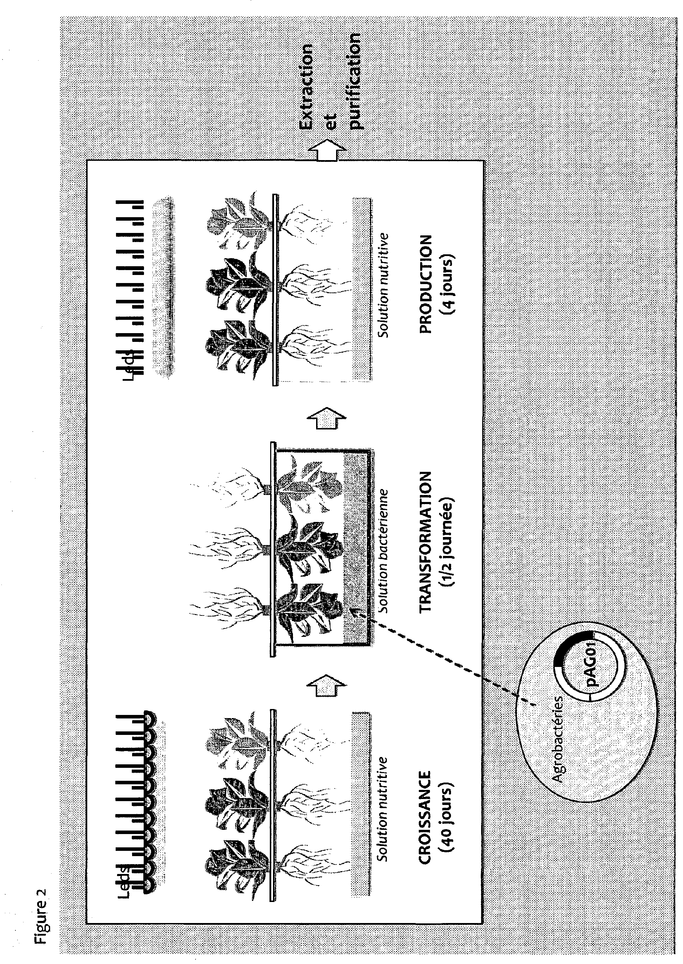

L'agroinfiltration des parties aériennes des plantes de tabac *N. benthamiana*, est réalisée sous vide. De préférence, on utilise un caisson d'étanchéité, qui possède un système de vide par effet Venturi. Typiquement, le caisson contient la culture d'agrobactéries et après retournement des plateaux flottants sur lesquels les plantes sont cultivées en hydroponie, ces dernières sont immergées, tête en bas, dans la suspension bactérienne. Ce procédé est illustré dans la figure 2. Il permet d'infiltrer simultanément toutes les plantes cultivées sur un même plateau flottant.

Selon un premier mode de réalisation, l'agroinfiltration est réalisée par une étape de mise sous vide des plantes pendant 2 minutes.
De préférence, selon un second mode de réalisation, l'agroinfiltration est réalisée en trois étapes (processus séquentiel) :
1) mise sous vide, de préférence à -0,8 bar pendant 2 minutes,
2) rupture du vide, et retour à la pression atmosphérique de préférence pendant 30 secondes, puis
3) mise sous vide, de préférence à -0,8 bar pendant 2 minutes, suivie du retour à la pression atmosphérique.
Cette technique d'agroinfiltration est rapide (durée totale inférieure à 5 minutes), efficace, et facile à automatiser.

Parmi les agrobactéries utilisables selon l'invention, on peut citer de préférence, les souches LBA4404, GV3101, EHA 101/105 ou C58.

De préférence, les agrobactéries sont utilisées pour l'infiltration à une concentration définie par une DO600 comprise entre 0,7 et 1,0, dans une solution comprenant 10 mM de Mes (Acide 2-morpholino éthanesulfonique), qui peut éventuellement être substitué par du MOPS (acide 3-(N-morpholinopropanesulfonique), 10mM de MgCl2 et 100µM d'acétosyringone.

A la fin de l'étape b) d'agroinfiltration, le procédé comprend une étape c) de remise en culture des plantes, dans les mêmes conditions que pour l'étape a).
Les plantes sont typiquement égouttées tête en bas pendant 15 minutes, puis remises en culture dans les conditions décrites pour l'étape a), idéalement en assurant une brumisation fréquente de ces dernières pendant les 6 premières heures de culture qui suivent l'agroinfiltation. Alternativement, les plantes sont directement remises en culture dans les conditions décrites pour l'étape a).

Enfin, le procédé selon l'invention comprend une étape d) d'extraction et de purification de la protéine recombinante produite après l'agroinfiltration à l'étape c).
La collecte de la biomasse végétale est effectuée 4 à 5 jours après la mise en culture des plantes suivant l' agroinfiltration.
Après le broyage et l'extraction des protéines à partir des parties aériennes des plantes, la protéine recombinante est purifiée. Les techniques d'extraction et de purification connues de l'art antérieur peuvent être mises en œuvre dans cette étape. De préférence, si la protéine recombinante comprend une séquence « tag histidine », elle est purifiée par chromatographie colonne de nickel immobilisé (IMAC), suivie d'une étape de tamisage moléculaire. La séquence tag utilisée pour la purification peut ensuite être clivée du produit final.

L'invention va maintenant être exemplifiée à l'aide des exemples qui suivent, qui ne sont pas limitatifs.

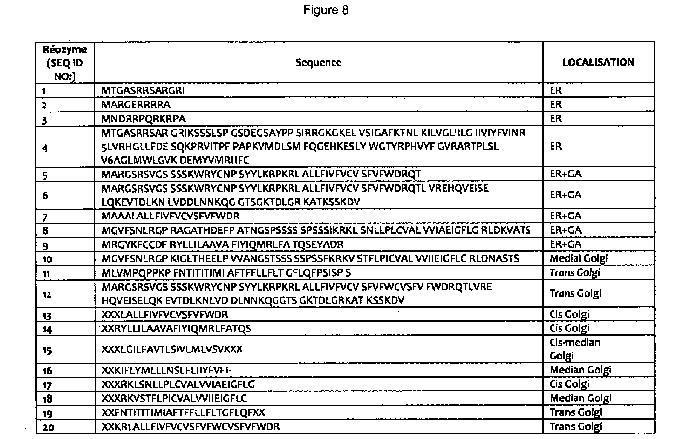

Les légendes des figures sont les suivantes :
Tableau 1 (Figure 8) : Séquences Reozyme™ utilisées pour l'expression ciblée d'allergènes recombinants et compartiment subcellulaire de stockage des allergènes lorsqu'ils sont produits en fusion avec ces peptides d'adressage. ER : réticulum endoplasmique ; GA : Appareil de Golgi.
Figure 1 : Le T-DNA du vecteur pAG01 est constitué des deux séquences bordantes du T-DNA des agrobactéries (RB et LB), et de trois cassettes d'expression permettant l'expression d'inhibiteur de silencing (cassette 1), d'une protéine recombinante de préférence un allergène (cassette 2) et d'une enzyme conférant une résistance à un antibiotique de sélection ou d'une enzyme de maturation des protéines (cassette 3).
Figure 2 : La plateforme développée selon l'invention associe des étapes et des outils originaux qui permettent la production en masse et à bas coût d'allergènes recombinants d'une très haute qualité inégalée. L'adaptabilité et la rapidité de production, puisque 4 à 5 jours sont suffisants pour passer du gène à la protéine, sont également des caractéristiques de cette plateforme de production.

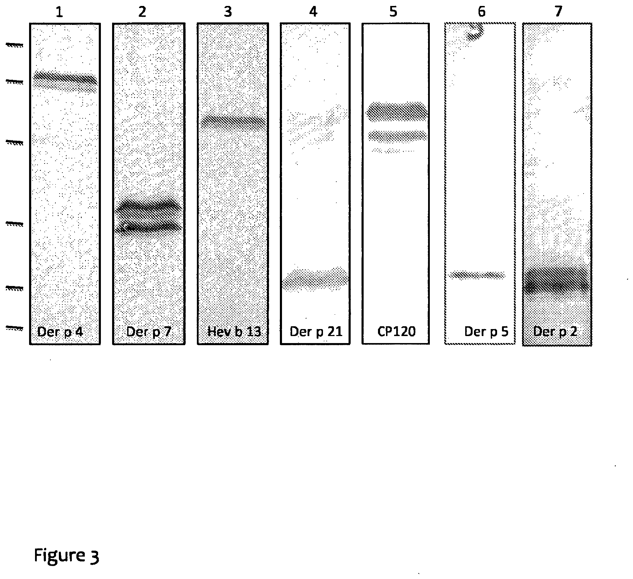

Figure 3 : Production d'allergènes majeurs complexes de l'acarien *Dermatophagoides pteronyssinus* : Der p 4 (piste 1) ; Der p 7 (piste 2); Der p 21 (piste 4); Der p 5 (piste 6) et Der p 2 (piste 7) ; l'un des allergènes majeurs de latex : Hev b 13 (piste 3) et un allergène de moisissure : CP120 (piste 5).

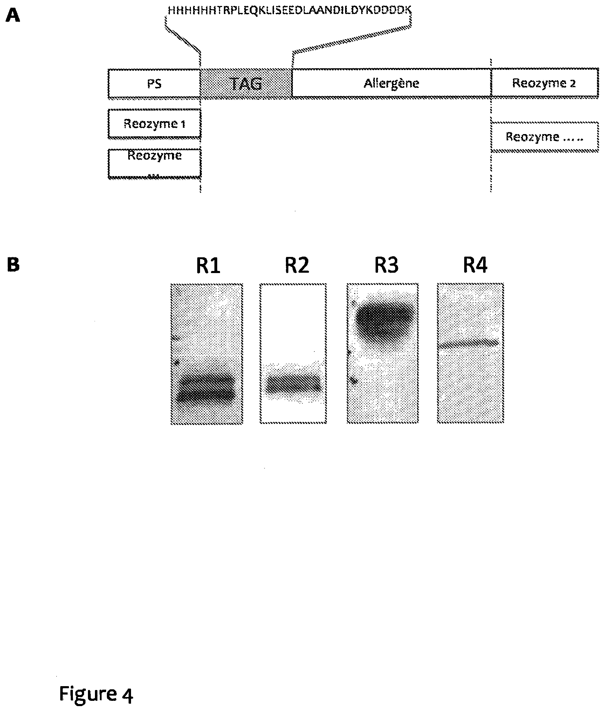

Figure 4 : Illustration schématique des cassettes d'expression utilisées (panneau A). Dans le panneau B, des analyses par Western blot illustrent les différences de qualité de l'allergène Der p 2 produit en fusion avec différents signaux Reozyme™. L'allergène est produit sous une forme hétérogène et présente une masse moléculaire non conforme lorsque les signaux Reozyme™ R1, R2 et R3 sont utilisés. En revanche, lorsque le signal R4 est utilisé, l'allergène recombinant est homogène et présente une masse moléculaire identique à celle de l'allergène naturel.

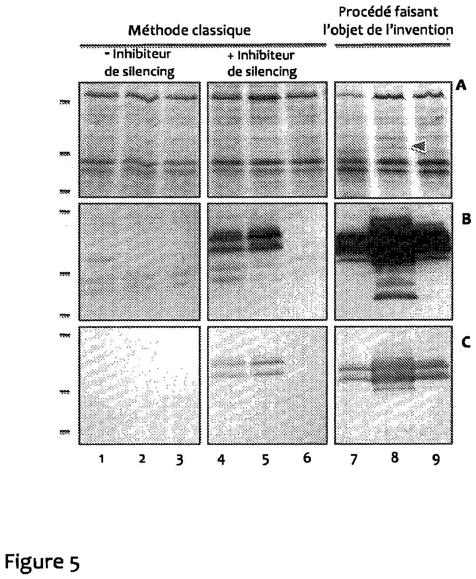

Figure 5 : Le panneau A présente l'analyse par SDS-PAGE des protéines totales extraites de chaque plante. L'analyse de ces extraits par Western blot (panneaux B et C) illustre les rendements de production pour une même protéine d'intérêt, lorsque les plantes sont cultivées soit dans des conditions standard (culture en pot + éclairage par lampe à incandescence) (pistes 1-6, les différentes pistes correspondent à différents évènements de tranformation), soit cultivées en aéroponie + éclairage LEDs (pistes 7-9). Panneau B : révélation chromogénique ; panneau C : révélation par chimioluminescence.

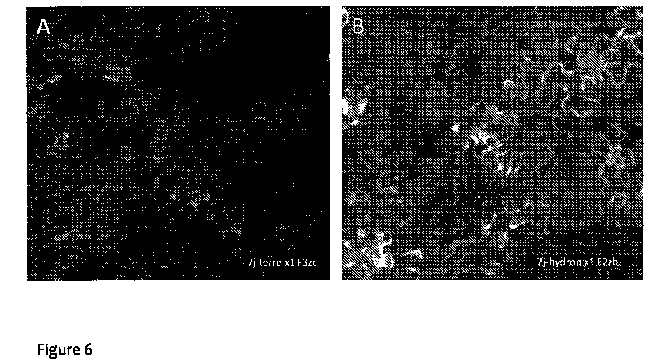

Figure 6 : Comparaison de l'expression de la GFP : 1) lorsque l'agroinfiltration est effectuée selon le protocole décrit dans l'invention (panneau B), ou 2) lorsque l'infiltration est effectuée selon une méthode d'infiltration classique (panneau A).

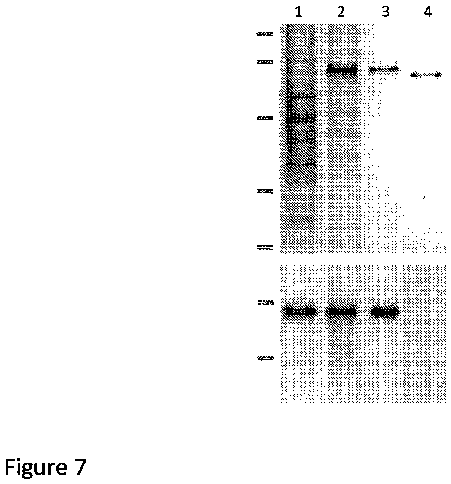

Figure 7 : Analyse par SDS-PAGE et Western Blot des étapes de purification de l'allergène Der p 4.
   Piste 1 : Extrait protéique total.
   Piste 2 : Der p 4 purifié sur la colonne de nickel immobilisé (IMAC) et élué en présence de 50mM d'imidazole
   Piste 3 : Der p 4 purifié par tamisage moléculaire après l'étape IMAC.
   Piste 4 : Der p 4 purifié et dont le tag a été clivé in vitro.
   Panneau supérieur : Analyse de Der p 4 par SDS-PAGE suivie d'une coloration des protéines par le bleu de Coomassie dans le gel.
   Panneau inférieur : Analyse de Der p 4 par SDS-PAGE suivi de western blotting et immunodétection sur l'empreinte avec un immunosérum spécifique du tag de purification.

### Exemple 1: Production standardisée d'antigènes complexes

Afin de valider le procédé décrit ici, des ADNc codant des allergènes complexes d'acariens, d'arbre ou de moisissures ont été clonés dans le vecteur pAG01. Ces vecteurs ont été ensuite introduits dans les agrobactéries (souche LBA4404) en vue de l'expression transitoire dans N. *benthamiana.*

Les plantes *N. benthamiana* ont été cultivées selon le procédé décrit ci-après : les graines sont semées en terre et cultivées pendant au plus 45 jours sur ce substrat. De préférence, les plantules issues de ces graines se développent durant 15 jours dans ce substrat (et sous éclairage LEDs), avant d'être transférées dans des bacs pour la culture hydroponique sur des flotteurs libres. Les plantes sont alors cultivées 25 jours dans ces conditions en présence de nutriments et d'oligoéléments et sous éclairage LEDs. Dans une alternative à ce protocole, l'utilisation de graines enrobées permet le semis direct des plantes de *N.benthamiana* sur les plateaux flottants. Dans ces conditions, la germination et la culture des plantes ont lieu en conditions d'hydroponie.

Après 40 jours de culture, les plantes maintenues sur les flotteurs sont transférées dans un caisson étanche en vue de leur transfection. Afin de faire pénétrer les agrobactéries porteuses du vecteur binaire, la partie aérienne des plantes est immergée (flotteur retourné) dans la solution d'agrobactéries dont la concentration correspond à une DO 600 : 0,7. La transfection est réalisée sous vide dans le caisson étanche par effet venturi selon le protocole suivant : 2min sous vide (-0,8 Bar), retour à la normale, puis de nouveau 2min sous vide (-0,8 Bar). Les flotteurs sont ensuite placés sur des supports (plantes tête en bas) durant 10 à 15 min, afin que les plantes s'égouttent. Puis les plantes, toujours maintenues sur les plateaux flottants sur lesquels elles sont cultivées, sont replacées dans les bacs de culture pour 4 jours, idéalement en assurant une brumisation fréquente de ces dernières pendant les 6 premières heures de culture qui suivent l'agroinfiltation.

Au terme de ces 4 jours, les parties aériennes des plantes exprimant les différents allergènes sont récoltées. Les protéines sont extraites dans un tampon dénaturant puis analysées en SDS-PAGE et/ou Western blotting à l'aide d'un anticorps dirigé contre l'épitope FLAG.

La figure 3 illustre les résultats obtenus. La méthode selon l'invention permet la production d'allergènes majeurs complexes.

### Exemple 2: le procédé selon l'invention permet un contrôle de la qualité des allergènes

Afin de contrôler la maturation et l'homogénéité des allergènes recombinants, différents signaux (R1, R2, R3 et R4) ont été fusionnés à l'allergène d'intérêt, puis la protéine de fusion a été clonée dans le vecteur pAG01. Ces vecteurs ont été ensuite introduits dans les agrobactéries (souche LBA4404) en vue de l'expression transitoire dans *N. benthamiana.*

Les plantes *N. benthamiana* ont été cultivées selon le procédé décrit ci-après : les graines sont semées en terre et les plantules issues de ces graines se développent durant 15 jours dans ce substrat (éclairage LEDs) avant d'être transférées sur des flotteurs libres. Les plantes sont alors cultivées 25 jours en hydroponie sur flotteurs libres.

Après 40 jours de culture dans ces conditions, les plantes maintenues sur les plateaux flottants de culture sont transférées dans un caisson étanche en vue de leur transfection. Afin de faire pénétrer les agrobactéries porteuses du vecteur binaire, les parties aériennes des plantes sont immergées (flotteur retourné) dans la solution d'agrobactéries. La transfection est réalisée sous vide dans le caisson étanche par effet venturi selon le protocole suivant : 2min sous vide (-0,8 bar), retour à la normale, puis de nouveau 2min sous vide (-0,8 Bar). Les flotteurs sont ensuite placés sur des supports (plantes tête en bas) durant 10 à 15 min afin que les plantes s'égouttent. Puis les plantes, toujours maintenues sur les flotteurs, sont replacées dans les bacs de culture pour 4 jours.

Au terme de ces 4 jours, les plantes exprimant les différents allergènes sont récoltées. Les protéines sont extraites par broyage des parties aériennes dans un tampon dénaturant puis analysées en SDS-PAGE et/ou Western blotting à l'aide d'un anticorps dirigé contre l'épitope FLAG.

La figure 4 présente une illustration schématique des cassettes d'expression utilisées (panneau A). Elle montre également, à travers l'exemple de l'allergène d'acarien Der p 2, les avantages qualitatifs liés à l'utilisation des signaux Reozyme™. En effet, dans le panneau B, des analyses par Western blot illustrent les différences de qualité de l'allergène Der p 2 produit en fusion avec différents signaux Reozyme™. L'allergène est produit sous une forme hétérogène et présente une masse moléculaire non conforme lorsque les signaux Reozyme™ R1, R2 et R3 sont utilisés. En revanche, lorsque le signal R4 est utilisé, l'allergène recombinant est homogène et présente une masse moléculaire identique à celle de l'allergène naturel.

### Exemple 3: Le procédé selon l'invention permet un rendement plus élevé

Pour cet exemple, nous avons comparé l'utilisation du vecteur pAG01 couplé à l'utilisation du procédé décrit dans l'invention à l'utilisation d'un vecteur binaire (-/+ inhibiteur de silencing) couplé à des méthodes classiques de transfection décrites par exemple dans Medrano *et al* (2009).

L'ADNc codant l'allergène Der p 7 de *Dermatophagoides pteronyssinus* a été cloné soit dans le vecteur pAG01, soit dans le vecteur pBI121. Ces vecteurs ont été ensuite introduits dans les agrobactéries (souche LBA4404) en vue de l'expression transitoire dans *N. benthamiana.*

Puis les souches d'agrobactéries ont été utilisées pour transfecter des plantes cultivées soit en hydroponie sur plateaux flottants tel que décrit dans les exemples 1 et 2, soit cultivées en terre puis infiltrées sous vide selon un protocole classique tel que publié dans Pogue *et al* (2010).

Comme l'illustre la figure 5, les conditions de cultures des plantes décrites dans le procédé, ainsi que l'utilisation du vecteur pAG01, permettent des rendements plus élevés en allergènes recombinants que ceux observés avec les conditions généralement utilisées pour l'expression transitoire.

Il apparaît clairement figure 5 que les rendements du procédé selon l'invention sont largement supérieurs à ceux obtenus avec un procédé classique. De plus, les inventeurs mettent en évidence une meilleure homogénéité des différents événements de transformation, comme l'illustrent les pistes 7 à 9 comparées aux pistes 4 à 6.

Les niveaux d'expression plus élevés observés pour Der p 7 s'expliquent en partie par les conditions d'agroinfiltration selon le procédé de l'invention. En effet, comme l'illustre la figure 6, l'infiltration des tissus foliaires est plus efficace tel qu'il est décrit dans le procédé.. Dans cette figure, les inventeurs ont comparé l'expression de la GFP : 1) lorsque l'agroinfiltration est effectuée selon le protocole décrit dans l'invention (panneau B), ou 2) lorsque l'infiltration est effectuée selon une méthode d'infiltration classique (panneau A) décrite par exemple dans Medrano et al (2009).

### Exemple 4: Le procédé selon l'invention permet une purification aisée

Les feuilles des plantes de *Nicotiana benthamiana* sont récoltées, et les protéines sont extraites par broyage de ce matériel végétal dans un tampon phosphate complété en NaCl (0,1 M), pH 7,5. Apres une filtration rapide, l'extrait est déposé sur une colonne de nickel immobilisé. Les protéines de l'extrait ne présentant pas d'affinité pour la matrice de chromatographie ne sont pas retenues sur la colonne. En revanche, les allergènes recombinants produits selon le procédé présentent une étiquette hexa-histidine et sont retenus sur ce type de matrice. Après un lavage de la colonne destiné à éliminer les protéines contaminantes, les allergènes sont élués spécifiquement en présence de 50 mM imidazole dans le tampon phosphate.

Le procédé de production selon l'invention est flexible et facilement adaptable à la production de tout allergène d'intérêt. Ceci est vrai non seulement pour les techniques de culture, de clonage, d'agroinfiltration, d'extraction, mais aussi pour la purification.

En effet, grâce à la fusion d'un tag, la purification des allergènes recombinants est standardisée. Ceci est illustré dans la figure 7, qui présente l'analyse par SDS-PAGE et western blotting des étapes de purification de l'allergène Der p 4 produit comme décrit dans l'invention. Cette analyse illustre le procédé de purification de cet allergène en deux étapes de chromatographie :
1) affinité sur colonne de nickel immobilisé (IMAC) et 2) tamisage moléculaire.
   Piste 1 : Extrait protéique total.
   Piste 2 : Der p 4 purifié sur la colonne de nickel immobilisé (IMAC) et élué en présence de 50mM d'imidazole
   Piste 3 : Der p 4 purifié par tamisage moléculaire après l'étape IMAC.
   Piste 4 : Der p 4 purifié et dont le tag a été clivé in vitro.
   Panneau supérieur : Analyse de Der p 4 par SDS-PAGE suivie d'une coloration des protéines par le bleu de Coomassie dans le gel.
   Panneau inférieur : Analyse de Der p 4 par SDS-PAGE suivi de western blotting et immunodétection sur l'empreinte avec un immunosérum spécifique du tag de purification.

## Revendications

1. Procédé de production d'un allergène recombinant dans une plante de tabac *Nicotiana benthamiana*, comprenant les étapes suivantes :
a) la culture de la plante en aéroponie ou en hydroponie et sous éclairage LEDs, puis
b) l'agroinfiltration de la plante obtenue en a), sous vide, par des agrobactéries comprenant un vecteur d'expression comprenant un fragment d'ADN codant pour l'allergène recombinant, puis
c) la remise en culture des plantes après l'étape b), dans les mêmes conditions que pour l'étape a), puis
d) l'extraction et la purification de l'allergène recombinant à partir des parties aériennes des plantes produites à l'étape c).

2. Procédé selon la revendication 1, **caractérisé en ce que** l'allergène recombinant est un allergène recombinant d'acarien.

3. Procédé selon la revendication 1 ou 2, **caractérisé en ce que** l'allergène recombinant est choisi parmi les *allergènes de Dermatophagoides farinae*, les allergènes de *Dermatophagoides pteronyssinus,* les allergènes de *Euroglyphus manei*, les allergènes *d'Acarus siro,* les allergènes de *Blomia tropicalis*, les allergènes de blattes, les allergènes de pollens d'arbres ou de graminées, les allergènes d'animaux, les allergènes de moisissures, les allergènes du latex d'hévéa et les allergènes responsables d'allergies alimentaires.

4. Procédé selon l'une des revendications 1 à 3, **caractérisé en ce que** l'allergène recombinant est choisi parmi Der f 10, Der f 11, Der f 13, Der f 14, Der f 15, Der f 16, Der f 17, Der f 18, Der f 2, Der f 2.0101, Der f 2.0102, Der f 2.0103, Der f 2.0104, Der f 2.0105, Der f 2.0106, Der f 2.0107, Der f 2.0108, Der f 2.0109, Der f 2.0110, Der f 2.0111, Der f 2.0112, Der f 2.0113, Der f 2.0114, Der f 2.0115, Der f 2.0116, Der f 2.0117, Der f20, Der f 3, Der f 4, Der f 5, Der f 6, Der f 7, Der f 8, Der f 9 et Der f HSP70.

5. Procédé selon l'une des revendications 1 à 3, **caractérisé en ce que** l'allergène recombinant est choisi parmi Der p 10, Der p 11, Der p 14, Der p 15, Der p 18, Der p 2, Der p 2.0101, Der p 2.0102, Der p 2.0103, Der p 2.0104, Der p 2.0105, Der p 2.0106, Der p 2.0107, Der p 2.0108, Der p 2.0109, Der p 2.0110, Der p 2.0111, Der p 2.0112, Der p 2.0113, Der p 20, Der p 21, Der p 3, Der p 4, Der p 5, Der p 6, Der p 7, Der p 8 et Der p 9.

6. Procédé selon l'une des revendications 1 à 3, **caractérisé en ce que** l'allergène recombinant est choisi parmi Blo t 1, Blo t 5, Blo t 9, Blo t 10, Blo t 12 et Blo t 21

7. Procédé selon l'une des revendications 1 à 6, **caractérisé en ce que** l'agroinfiltration est réalisée sous vide par effet Venturi.

8. Procédé selon la revendication 7, **caractérisé en ce que** l'agroinfiltration est réalisée soit par une étape de mise sous vide de la plante pendant 2 minutes, soit par mise sous vide, de préférence à - 0,8 bar pendant 2 minutes, puis rupture du vide et retour à la pression atmosphérique, de préférence pendant 30 secondes, puis mise sous vide, de préférence à - 0,8 bar pendant 2 minutes, suivie enfin du retour à la pression atmosphérique.

## Patentansprüche

1. Verfahren zur Herstellung eines rekombinanten Allergens in einer *Nicotiana benthamiana* Tabakpflanze, umfassend die folgenden Schritte:
a) Aeroponisches oder hydroponisches Züchten der Pflanze unter Beleuchtung durch LEDs, anschließend
b) Agroinfiltration der in a) erhaltenen Pflanze unter Vakuum mit Agrobakterien, die einen Expressionsvektor mit einem DNA-Fragment, das für das rekombinante Allergen kodiert, umfassen, anschließend
c) Weiterzüchten der Pflanzen nach dem Schritt b) unter den gleichen Bedingungen wie in Schritt a), anschließend
d) Extrahieren und Reinigen des rekombinanten Allergens aus den oberirdischen Teilen der in Schritt c) hergestellten Pflanzen.

2. Verfahren gemäß Anspruch 1, **dadurch gekennzeichnet, dass** das rekombinante Allergen ein rekombinantes Milbenallergen ist.

3. Verfahren gemäß Anspruch 1 oder 2, **dadurch gekennzeichnet, dass** das rekombinante Allergen ausgewählt ist aus Allergenen von *Dermatophagoides farinae,* Allergenen von *Dermatophagoides pteronyssinus,* Allergenen von *Euroglyphus manei,* Allergenen von *Acarus siro,* Allergenen von *Blomia tropicalis,* Schabenallergenen, Baum- oder Gräserpollenallergenen, Tierallergenen, Schimmelpilzallergenen, Kautschuklatexallergenen und Allergenen, die für Nahrungsmittelallergien verantwortlich sind.

4. Verfahren gemäß einem der Ansprüche 1 bis 3, **dadurch gekennzeichnet, dass** das rekombinante Allergen ausgewählt ist aus Der f 10, Der f 11, Der f 13, Der f 14, Der f 15, Der f 16, Der f 17, Der f 18, Der f 2, Der f 2.0101, Der f 2.0102, Der f 2.0103, Der f 2.0104, Der f 2.0105, Der f 2.0106, Der f 2.0107, Der f 2.0108, Der f 2.0109, Der f 2.0110, Der f 2.0111, Der f 2.0112, Der f 2.0113, Der f 2.0114, Der f 2.0115, Der f 2.0116, Der f 2.0117, Der f 20, Der f 3, Der f 4, Der f 5, Der f 6, Der f 7, Der f 8, Der f 9 und Der f HSP70.

5. Verfahren gemäß einem der Ansprüche 1 bis 3, **dadurch gekennzeichnet, dass** das rekombinante Allergen ausgewählt ist aus Der p 10, Der p 11, Der p 14, Der p 15, Der p 18, Der p 2, Der p 2.0101, Der p 2.0102, Der p 2.0103, Der p 2.0104, Der p 2.0105, Der p 2.0106, Der p 2.0107, Der p 2.0108, Der p 2.0109, Der p 2.0110, Der p 2.0111, Der p 2.0112, Der p 2.0113, Der p 20, Der p 21, Der p 3, Der p 4, Der p 5, Der p 6, Der p 7, Der p 8 und Der p 9.

6. Verfahren gemäß einem der Ansprüche 1 bis 3, **dadurch gekennzeichnet, dass** das rekombinante Allergen ausgewählt ist aus Blo t 1, Blo t 5, Blo t 9, Blo t 10, Blo t 12 und Blo t 21.

7. Verfahren gemäß einem der Ansprüche 1 bis 6, **dadurch gekennzeichnet, dass** die Agroinfiltration unter Vakuum unter Verwendung des Venturi-Effekts durchgeführt wird.

8. Verfahren gemäß Anspruch 7, **dadurch gekennzeichnet, dass** die Agroinfiltration durchgeführt wird, indem die Pflanze entweder in einem Schritt für 2 Minuten unter Vakuum gesetzt wird, oder indem sie unter Vakuum gesetzt wird, vorzugsweise bei - 0,8 bar für 2 Minuten, anschließend das Vakuum unterbrochen und zum Atmosphärendruck zurückgekehrt wird, vorzugsweise für 30 Sekunden, sie anschließend unter Vakuum gesetzt wird, vorzugsweise bei - 0,8 Bar für 2 Minuten, und schließlich zum Atmosphärendruck zurückgekehrt wird.

## Claims

1. Method for producing a recombinant allergen in a *Nicotiana benthamiana* tobacco plant, comprising the steps of:
a) growing the plant aeroponically or hydroponically under LED lighting, then
b) agroinfiltrating the plant obtained in a) under vacuum with agrobacteria comprising an expression vector comprising a DNA fragment coding for the recombinant allergen, then
c) returning the plants to growth after step b), under the same conditions as for step a), then
d) extracting and purifying the recombinant allergen from the aerial parts of the plants produced in step c).

2. Method according to claim 1, **characterised in that** the recombinant allergen is a recombinant mite allergen.

3. Method according to claim 1 or 2, **characterised in that** the recombinant allergen is chosen from the group consisting of the allergens of *Dermatophagoides farinae,* the allergens of *Dermatophagoides pteronyssinus,* the allergens of *Euroglyphus maynei*, the allergens of *Acarus siro*, the allergens of *Blomia tropicalis*, cockroach allergens, tree pollen allergens or grass pollen allergens, animal allergens, mould allergens, hevea latex allergens and allergens responsible for food allergies.

4. Method according to one of claims 1 to 3, **characterised in that** the recombinant allergen is chosen from the group consisting of Der f 10, Der f 11, Der f 13, Der f 14, Der f 15, Der f 16, Der f 17, Der f 18, Der f 2, Der f 2.0101, Der f 2.0102, Der f 2.0103, Der f 2.0104, Der f 2.0105, Der f 2.0106, Der f 2.0107, Der f 2.0108, Der f 2.0109, Der f 2.0110, Der f 2.0111, Der f 2.0112, Der f 2.0113, Der f 2.0114, Der f 2.0115, Der f 2.0116, Der f 2.0117, Der f20, Der f 3, Der f 4, Der f 5, Der f 6, Der f 7, Der f 8, Der f 9 and Der f HSP70.

5. Method according to one of claims 1 to 3, **characterised in that** the recombinant allergen is chosen from the group consisting of Der p 10, Der p 11, Der p 14, Der p 15, Der p 18, Der p 2, Der p 2.0101, Der p 2.0102, Der p 2.0103, Der p 2.0104, Der p 2.0105, Der p 2.0106, Der p 2.0107, Der p 2.0108, Der p 2.0109, Der p 2.0110, Der p 2.0111, Der p 2.0112, Der p 2.0113, Der p 20, Der p 21, Der p 3, Der p 4, Der p 5, Der p 6, Der p 7, Der p 8 and Der p 9.

6. Method according to one of claims 1 to 3, **characterised in that** the recombinant allergen is chosen from the group consisting of Blo t 1, Blo t 5, Blo t 9, Blo t 10, Blo t 12 and Blot 21.

7. Method according to one of claims 1 to 6, **characterised in that** the agroinfiltration is carried out under vacuum by the Venturi effect.

8. Method according to claim 7, **characterised in that** the agroinfiltration is carried out either by a step of putting the plant under vacuum for 2 minutes, or by putting under vacuum, preferably at -0.8 bar for 2 minutes, then by breaking the vacuum and returning to atmospheric pressure, preferably for 30 seconds, then by putting under vacuum, preferably at -0.8 bar for 2 minutes, followed finally by returning to atmospheric pressure.
